# EUROPEAN PATENT APPLICATION

(11) **EP 4 653 525 A1**
(43) Date of publication of application: **26.11.2025**
(21) Application number: 24177181.5
(22) Date of filing: 21.05.2024
(51) Int. Cl.: C12N 9/12

(54) **ENGINEERED TERMINAL DEOXYNUCLEOTIDYL TRANSFERASE POLYMERASES**

(71) Applicant: Uab "Biomatter Designs", 09120 Vilnius (LT)
(72) Inventor: Burdulis, Andrius, Vilnius (LT); Demcenko, Taisija, Vilnius (LT); Valinskyte, Skaiste, Vilnius (LT); Zakrys, Linas, Vilnius (LT); Rokaitis, Irmantas, Vilnius (LT)
(74) Representative: AAA Law

(57) **Abstract**

The present invention provides an engineered terminal deoxynucleotidyl transferase (TdT) useful in template-independent polynucleotide synthesis, the terminal deoxynucleotidyl transferase having increased thermostability and/or increased TdT activity and/or increased protein solubility, as well as compositions comprising the said terminal deoxynucleotidyl transferase and methods comprising the use of said terminal deoxynucleotidyl transferase.

## Description

### FIELD OF INVENTION

The present invention is related to engineered terminal deoxynucleotidyl transferase (TdT) polypeptides, more specifically, to mutant versions of terminal deoxynucleotidyl transferase, including methods and compositions thereof.

### BACKGROUND OF INVENTION

Terminal deoxynucleotidyl transferase (TdT) is a template-independent DNA polymerase that elongates oligonucleotides in 5' to 3' direction, producing single-stranded DNA of various lengths. TdT is extensively used in molecular biology research and medicine sectors for various applications such as TUNEL (TdT dUTP nick end labeling) assays, where TdT is used for labeling of 3' termini in the double-strand DNA breaks generated during apoptosis, detection of low amounts of viral DNA or RNA, and in recent decades, TdT has been extensively improved, optimised and adapted to fit the rapidly growing need of template-independent polymerases suitable for enzyme based de novo DNA synthesis (Hoose et al., 2023). One major shortcoming of TdT polymerase is the potential inhibition of DNA elongation by the secondary structure formation of single-stranded DNA substrates. Most commercially available TdTs are active at up to -37°C temperature, but to efficiently inhibit the formation of DNA secondary structures, reaction temperature should be raised to ~60°C. Thus, a more thermostable TdT enzyme, with higher optimal reaction temperature of ~50-60°C would greatly improve the efficiency of all TdT-dependent processes and be highly beneficial to the quickly growing industry of enzymatic DNA synthesis. Moreover, during template-independent enzymatic oligonucleotide synthesis (TiEOS), usually reversibly blocked nucleotides are employed - NTPs are modified with a synthesis terminator group at the 3' position. This approach of enzyme-based DNA synthesis requires the ability of TdT enzymes to efficiently utilise 3' modified nucleotides as a substrate, but wild-type TdT polymerases incorporate such nucleotides with reduced efficiency when compared to unmodified NTPs. Low efficiency of 3' reversibly blocked nucleotide incorporation is especially detrimental when synthesising DNA fragments longer than 200 bp. Since optimization of wild-type TdT enzymes in order to increase the efficiency of modified NTPs incorporation may result in losses in protein stability and majority of natural TdT enzymes are derived from mesophilic organisms, such as, e.g. *Bos taurus,* a TdT variant with improved thermostability would be an ideal starting point for further enzyme evolution (Chua et al., 2020).

Thus, there are needs for terminal deoxynucleotidyl transferase having improved properties such as increased thermal stability, as well as improved or at least retained TdT activity and/or TdT protein solubility. Provided herein are terminal deoxynucleotidyl transferases and related methods and compositions that can solve these needs and/or provide other benefits.

### SUMMARY OF INVENTION

The present invention provides an engineered terminal deoxynucleotidyl transferase (TdT) useful in template-independent polynucleotide synthesis, the terminal deoxynucleotidyl transferase having increased thermostability and/or increased TdT activity and/or increased protein solubility as compared to the wild-type TdT. These engineered TdT polypeptides are described, below, in the detailed description of the invention.

Accordingly, the present invention provides the following:
In a first aspect the present invention provides an engineered terminal deoxynucleotidyl transferase (TdT) comprising a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1. In some embodiments, the engineered terminal deoxynucleotidyl transferase comprises amino acid substitutions at positions corresponding to residues S59, W200 and M350. In some embodiments, the engineered terminal deoxynucleotidyl transferase comprises amino acid substitutions at positions corresponding to residues S59, W200, M350, K156 and M316. In other embodiments, the engineered terminal deoxynucleotidyl transferase comprises at least two amino acid substitutions at positions corresponding to residues selected from S59, W200 and M350. In yet other embodiments, the engineered terminal deoxynucleotidyl transferase comprises at least two amino acid substitutions at positions corresponding to residues selected from S59, W200, M350, K156 and M316. In some embodiments, the engineered terminal deoxynucleotidyl transferase polypeptide sequence further has at least 60%, 70%, 80%, 85%, 90%, 91%, 92%, 93%, 94%, 95%, 96%, 97%, 98%, or 99% sequence identity to a sequence selected from SEQ ID NO: 2 to 26, or has at least 60%, 70%, 80%, 85%, 90%, 91%, 92%, 93%, 94%, 95%, 96%, 97%, 98%, or 99% sequence identity to a sequence selected from SEQ ID NO: 27 to 49.

In some embodiments, the engineered terminal deoxynucleotidyl transferase of the present invention comprises amino acid substitutions at positions corresponding to residues S59, W200 and M350, or at positions corresponding to residues S59, W200, M350, K156 and M316, wherein the transferase further comprises at least one amino acid substitution or amino acid substitution set at amino acid positions selected from Y44, H46, I47, N58, F61, Y68, M72, T84, I86, V101, C103, 1109, D111, E113, A119, S128, S143, E144, F147, M149, F151, S153, S155, S159, L174, C182, T211, E237, Q281, S296, I310, C317, K348, and A355, and/or any combinations thereof, wherein the amino acid positions are numbered with reference to SEQ ID NO: 1.

In a second aspect, the present invention provides an engineered terminal deoxynucleotidyl transferase comprising a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 2 to 26. In some embodiments, the engineered terminal deoxynucleotidyl transferase comprises a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 2 to 26. In some embodiments, the engineered terminal deoxynucleotidyl transferase comprises a polypeptide sequence selected from SEQ ID NO: 2-26.

In some embodiments, the present disclosure provides an engineered terminal deoxynucleotidyl transferase comprising a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 27 to 49. In some embodiments, the engineered terminal deoxynucleotidyl transferase comprises a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 27 to 49. In some embodiments, the engineered terminal deoxynucleotidyl transferase comprises a polypeptide sequence selected from SEQ ID NO: 27 to 49.

The present disclosure further provides compositions, reaction mixtures and/or kits comprising the engineered terminal deoxynucleotidyl transferase according to the present invention.

In a third aspect, a method for synthesizing a nucleic acid molecule without template, comprising a step of contacting a nucleic acid primer with at least one nucleotide and the engineered terminal deoxynucleotidyl transferase according to the present invention is provided. In some embodiments, the synthesis of the nucleic acid molecule is performed at temperature of at least 50°C, at least 60°C, or at least 70°C.

### BRIEF DESCRIPTION OF DRAWINGS

Fig. 1. Whole cell lysate (WCL) and soluble fraction (Sol. fr.) samples of engineered and wild-type (WT TdT) TdT polymerases analyzed in SDS-PAGE gels; M - protein molecular weight ladder. Arrows indicate target proteins. B2P5, B2P9, B2P15, B2P19, B2P26 in the Figure refer to R1281B2P5, R1281B2P9, R1281B2P15, R1281B2P19, R1281B2P26 TdT variants, respectively. The same applies to Figures 2 to 7.
Fig. 2. Purified engineered and wild-type (WT TdT) TdT polymerases analyzed in SDS-PAGE gels; M - protein molecular weight ladder. Arrows indicate purified proteins.
Fig. 3. Purification yields of engineered and wild-type (WT TdT) TdT polymerases in mg of obtained soluble protein per liter of bacterial expression culture.
Fig. 4. Melting temperature (Tm) values of engineered and wild-type (WT TdT) TdT polymerases
Fig. 5. Activity of purified engineered and wild-type (WT TdT) TdT polymerases measured by their ability to add dATP molecules to initiator primer at 22°C temperature. Fragment ladder denotes the addition of increasing number of nucleotides to the initiator primer.
Fig. 6. Activity of purified engineered and wild-type (WT TdT) TdT polymerases measured by their ability to add dATP molecules to initiator primer at 22°C temperature after incubations in 37, 47, 57 and 67°C temperatures. Fragment ladder denotes the addition of increasing number of nucleotides to the initiator primer.
Fig. 7. Activity of purified engineered and wild-type (WT TdT) TdT polymerases measured by their ability to add dATP molecules to initiator primer at 37, 50, 60 and 70°C temperatures. Fragment ladder denotes the addition of increasing number of nucleotides to the initiator primer.
Figs. 8 A through 8G show a multiple amino acid sequence alignment of the WT TdT (SEQ ID NO: 1) and examples of engineered TdT of the present invention (SEQ ID NO: 2 to 26), in which the positions corresponding to positions 59, 156, 200, 316 and 350 of SEQ ID NO: 1 are marked with an asterisk (*).

### DETAILED DESCRIPTION OF THE INVENTION

This description and exemplary embodiments should not be taken as limiting. For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

As used in this specification and the appended claims, the singular forms "a," "an," and "the," and any singular use of any word, include plural referents unless expressly and unequivocally limited to one referent. It is also noted that the term "comprising" is intended to be open and permits but does not require the inclusion of additional elements or steps. When the term "comprising" is used herein, the term "consisting of' is thus also encompassed and disclosed.

As used herein, "engineered", "modified" and "non-naturally occurring" when used with reference to a cell, nucleic acid, or polypeptide, refer to a material, or a material corresponding to the natural or native form of the material, that has been modified in a manner that would not otherwise exist in nature. Such "engineered" or "modified" sequences, for example, polypeptide sequences, are functional or working. By "functional" or "working" it is meant that they can perform their intended function, preferably to at least the same extent as any existing native counterparts. Optionally, an engineered sequence may have at least one improved function or property relative to any existing native counterparts.

"Percentage of sequence identity" and "percentage homology" are used interchangeably herein to refer to comparisons among polynucleotides or polypeptides, and are determined by comparing two optimally aligned sequences over a comparison window, wherein the portion of the polynucleotide or polypeptide sequence in the comparison window may comprise additions or deletions (i.e., gaps) as compared to the reference sequence for optimal alignment of the two sequences. The percentage may be calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. Alternatively, the percentage may be calculated by determining the number of positions at which either the identical nucleic acid base or amino acid residue occurs in both sequences or a nucleic acid base or amino acid residue is aligned with a gap to yield the number of matched positions, dividing the number of matched positions by the total number of positions in the window of comparison and multiplying the result by 100 to yield the percentage of sequence identity. Those of skill in the art appreciate that there are many established algorithms available to align two sequences. Optimal alignment of sequences for comparison can be conducted, e.g., by the local homology algorithm of Smith and Waterman (Smith and Waterman, Adv. Appl. Math., 2:482 [1981]), by the homology alignment algorithm of Needleman and Wunsch (Needleman and Wunsch, J. Mol. Biol., 48:443 [1970]), by the search for similarity method of Pearson and Lipman (Pearson and Lipman, Proc. Natl. Acad. Sci. USA 85:2444 [1988]), by computerized implementations of these algorithms (e.g., GAP, BESTFIT, FASTA, and TFASTA in the GCG Wisconsin Software Package), or by visual inspection, as known in the art. Examples of algorithms that are suitable for determining percent sequence identity and sequence similarity include, but are not limited to the BLAST and BLAST 2.0 algorithms, which are described by Altschul et al. (See, Altschul et al., J. Mol. Biol., 215: 403-410 [1990]; and Altschul et al., Nucl. Acids Res., 3389-3402 [1977], respectively). Software for performing BLAST analyses is publicly available through the National Center for Biotechnology Information website. This algorithm involves first identifying high scoring sequence pairs (HSPs) by identifying short words of length W in the query sequence, which either match or satisfy some positive-valued threshold score T when aligned with a word of the same length in a database sequence. T is referred to as, the neighborhood word score threshold (Altschul et al., supra). These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always >0) and N (penalty score for mismatching residues; always <0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative -scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4, and a comparison of both strands. For amino acid sequences, the BLASTP program uses as defaults a wordlength (W) of 3, an expectation (E) of 10, and the BLOSUM62 scoring matrix (See, Henikoff and Henikoff, Proc. Natl. Acad. Sci. USA 89: 10915 [1989]). Exemplary determination of sequence alignment and % sequence identity can employ the BESTFIT or GAP programs in the GCG Wisconsin Software package (Accelrys, Madison WI), using default parameters provided.

"Reference sequence" refers to a defined sequence used as a basis for a sequence comparison. A reference sequence may be a subset of a larger sequence, for example, a segment of a full-length gene or polypeptide sequence. Generally, a reference sequence is at least 20 nucleotide or amino acid residues in length, at least 25 residues in length, at least 50 residues in length, or the full length of the nucleic acid or polypeptide. Since two polynucleotides or polypeptides may each (1) comprise a sequence (i.e., a portion of the complete sequence) that is similar between the two sequences, and (2) may further comprise a sequence that is divergent between the two sequences, sequence comparisons between two (or more) polynucleotides or polypeptide are typically performed by comparing sequences of the two polynucleotides or polypeptides over a "comparison window" to identify and compare local regions of sequence similarity. In some embodiments, a "reference sequence" can be based on a primary amino acid sequence, where the reference sequence is a sequence that can have one or more changes in the primary sequence.

"Comparison window" refers to a conceptual segment of at least about 20 contiguous nucleotide positions or amino acids residues wherein a sequence may be compared to a reference sequence of at least 20 contiguous nucleotides or amino acids and wherein the portion of the sequence in the comparison window may comprise additions or deletions (i. e. , gaps) of 20 percent or less as compared to the reference sequence (which does not comprise additions or deletions) for optimal alignment of the two sequences. The comparison window can be longer than 20 contiguous residues, and includes, optionally 30, 40, 50, 100, or longer windows.

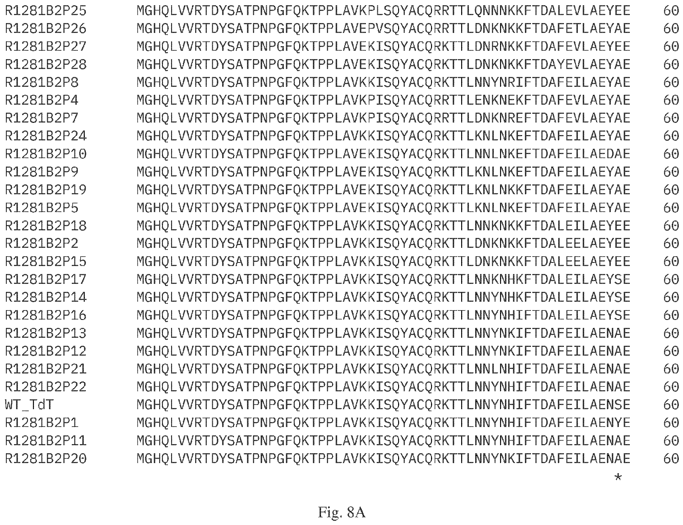

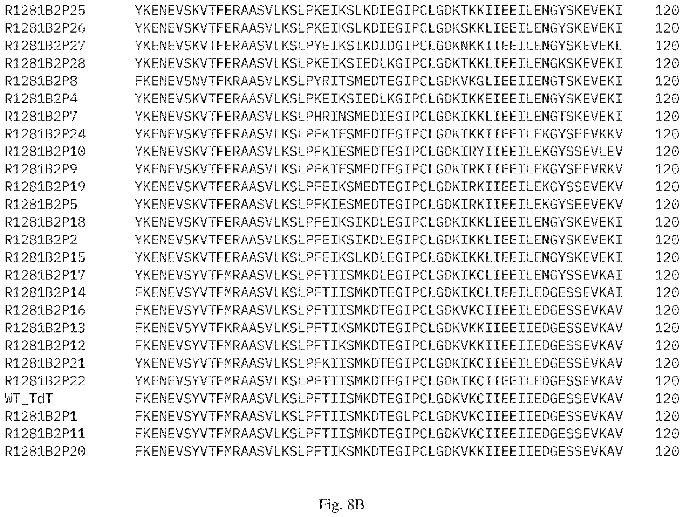

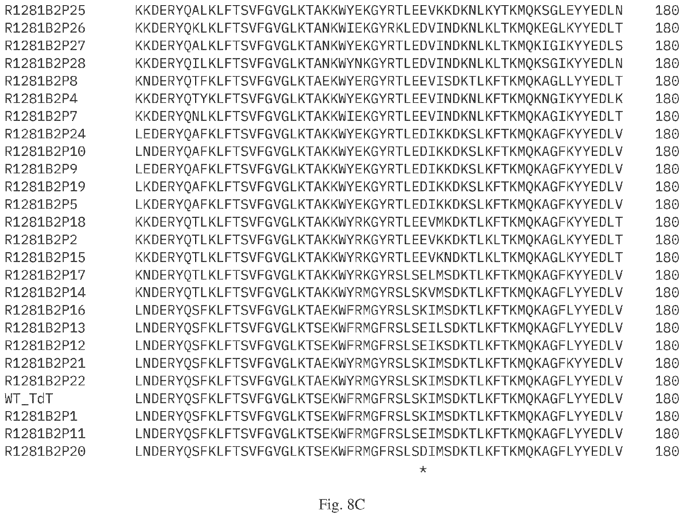

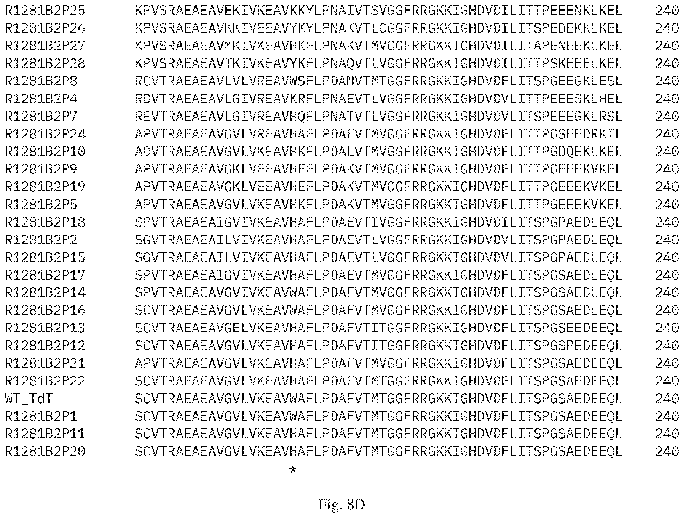

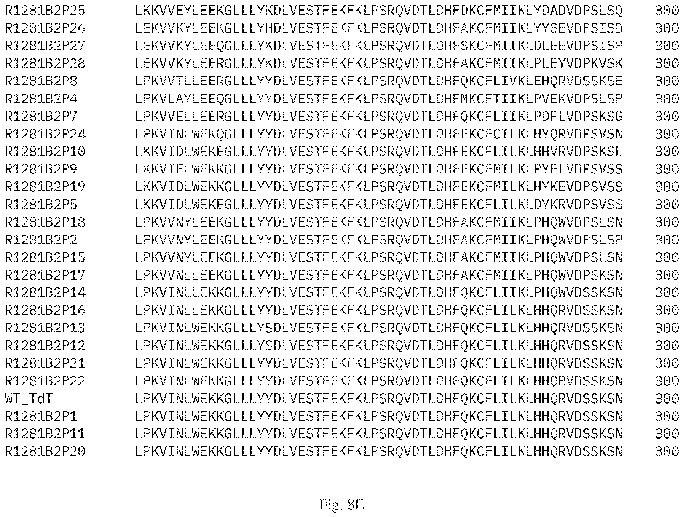

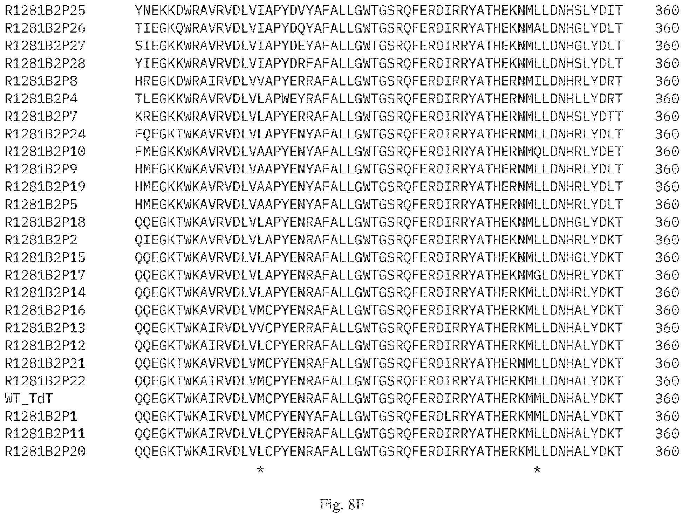

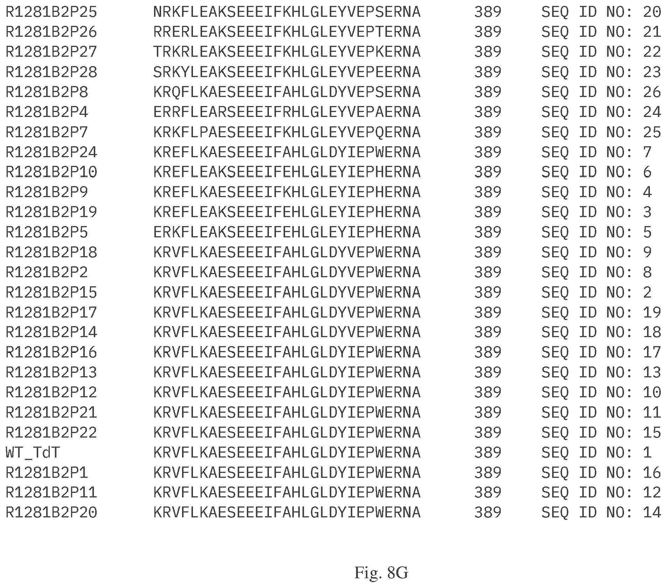

"Amino acid difference" or "residue difference" or "substitution" refers to a change in the amino acid residue at a position of a polypeptide sequence relative to the amino acid residue at a corresponding position in a reference sequence. The positions of amino acid differences generally are referred to herein as "Xn," where n refers to the corresponding position in the reference sequence upon which the residue difference is based. For example, a "residue difference at position S59 as compared to SEQ ID NO: 1" refers to a change of the amino acid residue at the polypeptide position corresponding to position 59 of SEQ ID NO: 1. Thus, if the reference polypeptide of SEQ ID NO: 1 has a serine at position 59, then a "residue difference at position S59 as compared to SEQ ID NO: 1" is an amino acid substitution of any residue other than serine at the position of the polypeptide corresponding to position 59 of SEQ ID NO: 1. As a nonlimiting example with reference of sequence alignment and identification of amino acid positions corresponding to the respective positions of the reference sequence, Figure 8 provides a multiple sequence alignment with some of the specific amino acid positions of SEQ ID NO: 1 and their corresponding amino acid positions in the amino acid sequences of exemplary engineered TdT sequences indicated.

"Protein solubility", as provided herein, in the context of recombinant protein expression, refers to the ability of a recombinantly produced protein to remain dissolved in the aqueous phase of the expression system, rather than forming insoluble aggregates or inclusion bodies. Achieving soluble expression of recombinant proteins is critical for downstream applications. Insoluble proteins typically require denaturation and refolding steps, which can be complex and even not practically applicable. Accordingly, protein that has low solubility or even insoluble in recombinant expression systems provides additional hurdles for its research and use. Therefore, maximizing protein solubility is one of the key steps in recombinant protein production.

Terminal deoxynucleotidyl transferase (TdT) is a template-independent polymerase. The "enzyme activity" of TdT refers to the ability of the TdT enzyme to catalyze the addition of natural or modified nucleotides to the 3' end of a nucleic acid without the need for a template nucleic acid, wherein the nucleic acid may be a DNA or an RNA molecule. Therefore, only a nucleic acid primer which can be termed "an initiator primer" and which has a 3'-OH, as well as at least one nucleotide is needed to initiate the nucleotide addition reaction. The nucleotide addition reaction conditions are well known to the skilled person in the art. When referring to "increased activity" of an engineered TdT as provided herein, it is meant that a the engineered TdT is capable to add more nucleotides to the nucleic acid primer under the same nucleic acid synthesis reaction conditions in comparison with a reference wild-type (WT) TdT (SEQ ID NO: 1) enzyme.

"Thermostability", as provided herein relates to the effect of temperature on the activity of enzymes. Typically, enzyme activity increases with temperature up to an optimum temperature. As the temperature is increased beyond the optimum temperature, enzyme activity diminishes due to the disruption of bonds, particularly hydrogen bonds, which maintain the secondary and tertiary structures of enzymes. Once the tertiary structure of the enzyme is lost, the enzyme is considered denatured and essentially, inactive. The temperature at which this occurs is the denaturation temperature. Enzyme improvement may include improving thermostability of the target sequence and more specifically, increasing the denaturation temperature such that the engineered enzyme retains activity (i.e. is stable) at a wider range of temperatures or exhibit improved activity at a desired temperature. A temperature at which a TdT such as a TdT from Bos taurus is typically used and remains active is from about 22°C to 37°C, however, in order to efficiently inhibit the formation of nucleic acid (e.g. DNA) secondary structures, a higher temperature of the TdT reaction is often needed. Enzyme improvement may further include increasing the melting temperature of the protein wherein the melting temperature may be defined as the temperature at which the free energy change of unfolding (AG) is zero and 50% of the population of protein molecules is in the folded state whilst 50% of the population of protein molecules is in the unfolded state. Examples of techniques used for measurement of melting temperature are circular dichroism (CD) spectroscopy, differential scanning calorimetry (DSC) and fluorescent thermal shift assay (TSA). TSA is a high-throughput method that measures thermal stability of the protein tertiary structure using a fluorescent protein- binding probe which detects protein aggregation.

### Engineered terminal deoxynucleotidyl transferases (TdT)

The present invention disclosure provides engineered terminal deoxynucleotidyl transferases (TdT), the amino acid sequences of which have been engineered by adding various amino acid substitutions in comparison to the reference TdT enzyme from *Bos taurus.*

It is known that TdT such as, e.g. TdT from *Bos taurus,* is composed of distinct domains from N-terminus to C-terminus that correspond to nuclear localization domain (NLS), BRCT-like domain, a linker and a catalytic domain. The reference protein WT TdT (SEQ ID NO: 1) as provided by the present disclosure comprises to the positions of amino acid sequence of 123-509 aa of the bovine TdT (Uniprot number: P06526), in particular, positions 3-389 aa of SEQ ID NO: 1 fully align with positions 123-509 of bovine TdT (Uniprot number: P06526). Accordingly, the reference sequence SEQ ID NO: 1 as provided herein comprises a linker and a catalytic domain and is capable of functioning as a terminal deoxynucleotidyl transferase. The TdT may be further truncated, and comprise an amino acid sequence that has an N-terminal truncation in reference to SEQ ID NO: 1. Various N-terminally truncated functionally active forms of TdT are known, for example, those identified in US7494797B2). The shortest one that comprises the TdT catalytic domain and has TdT activity corresponds to the amino acid sequence corresponding to amino acid positions 123 to 389 of the presently provided WT TdT (SEQ ID NO: 1). Such versions of TdT are referred to as "functional fragment", by which is meant that the fragment or derivative is a polypeptide having one or more of the biological properties of terminal deoxyribonucleotide transferase. Additions, deletions, substitutions and derivatizations of one or more of the amino acids of the polypeptide are contemplated so long as the modifications do not result in loss of functional activity of the fragment or derivative.

One of the other examples of functional fragment form of TdT may be a circularly permuted form of the polypeptide. As used herein, the term "circularly permuted" refers to an engineered or modified polynucleotide or polypeptide (i.e., a molecule having a linear primary structure), wherein the molecule comprises two terminal components (e.g., a 5' end and a 3' end, e.g. an N-terminus and a C-terminus, etc.) that are or have been joined together, either directly or indirectly such as, e.g., via a linker, to produce a substantially circular/circularized molecule, which, after initial circularization, is then opened at another location on the circularized molecule, which location is distinct from the location where two termini of the linear molecule were initially joined. The resulting molecule is a new substantially linear molecule with a second set of two termini that differ from the first set of two termini in the first joining (i.e., in the "original" or "native" or "parent" molecule). As provided herein circular permutation retains at least a portion of amino acid or nucleic acid sequence identity with that of the parent molecule, but in a rearranged order (i.e., sequence) relative to the parent, generating new termini (relative to those of the starting molecule). Circularly permuted proteins have been long known to be present in nature as well as can be made artificially (Bliven et al., PloS Comput Biol. 8(3), 2012). Circular permutation and processes to generate circularly permuted molecules are generally known to those of skill in the art. Circular permuted TdT versions are also known from the art, for example, from WO2023240202A1. Accordingly, as skilled person will be able to produce circularly permuted functional TdT from the engineered TdT variants provided herein. As an example, a version of an engineered TdT of the present invention comprising the amino acid sequence corresponding to amino acid positions 123 to 389 (i.e. a functional fragment) in reference to WT TdT (SEQ ID NO: 1) can be used to generate a circular permuted version of the engineered TdT.

As described further herein, the engineered terminal deoxynucleotidyl transferase (TdT) polypeptides of the present disclosure have amino acid substitutions as compared to the wild type TdT, are functionally active and exhibit one of more improved properties such as thermostability, enzyme activity, protein solubility as compared to a WT TdT. Various embodiments of such engineered TdT are provided herein.

In some embodiments, the present invention provides an engineered terminal deoxynucleotidyl transferase (TdT) comprising a polypeptide sequence having at least 60% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1, wherein the engineered terminal deoxynucleotidyl transferase has increased thermostability and/or increased TdT activity and/or increased protein solubility as compared to the WT TdT. Various forms of functional fragments of may be useful, including, for example, the amino acid sequence corresponding to amino acid positions 123 to 389 (i.e. a functional fragment) in reference to WT TdT (SEQ ID NO: 1). In some embodiments, a circularly permuted form of the engineered TdT comprising amino acid sequence corresponding to amino acid positions 123 to 389 in reference to WT TdT (SEQ ID NO: 1). In some embodiments, the engineered terminal deoxynucleotidyl transferase (TdT) comprises a polypeptide sequence having at least 70% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1. In some embodiments, the engineered terminal deoxynucleotidyl transferase (TdT) comprises a polypeptide sequence having at least 80% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1. In some embodiments, the engineered terminal deoxynucleotidyl transferase (TdT) comprises a polypeptide sequence having at least 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1.

In some embodiments, the engineered terminal deoxynucleotidyl transferase according to present invention comprises a polypeptide sequence having at least 60%, 70%, 80%, 90%, 91%, 92%, 93%, 94%, 95%, 96%, 97%, 98%, or 99% sequence identity to a sequence SEQ ID NO: 1, or a functional fragment thereof and comprises amino acid substitutions at positions corresponding to residues S59, W200 and M350, wherein the positions are numbered by reference to the amino acid sequence set forth in SEQ ID NO: 1, wherein the engineered terminal deoxynucleotidyl transferase has increased thermostability and/or increased TdT activity and/or increased protein solubility as compared to the WT TdT.

In some embodiments, the engineered terminal deoxynucleotidyl transferase according to present invention comprises a polypeptide sequence having at least 60%, 70%, 80%, 90%, 91%, 92%, 93%, 94%, 95%, 96%, 97%, 98%, or 99% sequence identity to a sequence SEQ ID NO: 1, or a functional fragment thereof and comprises amino acid substitutions at positions corresponding to residues S59, W200, M350, K156 and M316, wherein the positions are numbered by reference to the amino acid sequence set forth in SEQ ID NO: 1, wherein the engineered terminal deoxynucleotidyl transferase has increased thermostability and/or increased TdT activity and/or increased protein solubility as compared to the WT TdT.

In some embodiments, the engineered terminal deoxynucleotidyl transferase according to present invention comprises a polypeptide sequence having at least 60%, 70%, 80%, 90%, 91%, 92%, 93%, 94%, 95%, 96%, 97%, 98%, or 99% sequence identity to a sequence SEQ ID NO: 1, or a functional fragment thereof and comprises at least two amino acid substitutions at positions corresponding to residues selected from S59, W200 and M350, wherein the positions are numbered by reference to the amino acid sequence set forth in SEQ ID NO: 1, wherein the engineered terminal deoxynucleotidyl transferase has increased thermostability and/or increased TdT activity and/or increased protein solubility as compared to the WT TdT.

In some embodiments, the engineered terminal deoxynucleotidyl transferase according to present invention comprises a polypeptide sequence having at least 60%, 70%, 80%, 90%, 91%, 92%, 93%, 94%, 95%, 96%, 97%, 98%, or 99% sequence identity to a sequence SEQ ID NO: 1, or a functional fragment thereof and comprises at least two amino acid substitutions at positions corresponding to residues selected from S59, W200, M350, K156 and M316, wherein the positions are numbered by reference to the amino acid sequence set forth in SEQ ID NO: 1, wherein the engineered terminal deoxynucleotidyl transferase has increased thermostability and/or increased TdT activity and/or increased protein solubility as compared to the WT TdT. In some embodiments, the engineered terminal deoxynucleotidyl transferase comprises a substitution selected from the group consisting of S59A, S59E, S59Y.

In some embodiments, the engineered terminal deoxynucleotidyl transferase comprises a substitution selected from the group consisting of W200H, W200K, W200Y, preferably the substitution is W200H.

In some embodiments, the engineered terminal deoxynucleotidyl transferase comprises a substitution selected from the group consisting of M350L, M350Q, M350G, M350A, M350I, preferably the substitution is M350L.

In some embodiments, the engineered terminal deoxynucleotidyl transferase comprises a substitution selected from the group consisting of K156E, K156D.

In some embodiments, the engineered terminal deoxynucleotidyl transferase comprises a substitution selected from the group consisting of M316L, M316A, M316V, M316I.

In further embodiments, provided herein is an engineered terminal deoxynucleotidyl transferase having at least 60%, 70%, 80%, 85%, 90%, 91%, 92%, 93%, 94%, 95%, 96%, 97%, 98%, or 99% sequence identity to a sequence selected from SEQ ID NO: 2 to 26, wherein the engineered terminal deoxynucleotidyl transferase has increased thermostability and/or increased TdT activity and/or increased protein solubility as compared to the WT TdT. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to a sequence selected from SEQ ID NO: 2 to 9. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to a sequence selected from SEQ ID NO: 2 to 9. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to a sequence selected from SEQ ID NO: 10 to 19. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to a sequence selected from SEQ ID NO: 20 to 26. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 2. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 3. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 4. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 5. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 6. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 7. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 8. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 9. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 10. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 11. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 12. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 13. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 14. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 15. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 16. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 17. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 18. In some embodiments, the engineered terminal deoxynucleotidyl transferase has at least 60%, 70%, 80%, 85%, 90%, 91%, 92%, 93%, 94%, 95%, 96%, 97%, 98%, or 99% sequence identity to SEQ ID NO: 19.

In further embodiments, provided herein is an engineered terminal deoxynucleotidyl transferase having at least 60%, 70%, 80%, 85%, 90%, 91%, 92%, 93%, 94%, 95%, 96%, 97%, 98%, or 99% sequence identity to a sequence selected from SEQ ID NO: 27 to 49.

In some embodiments, the engineered terminal deoxynucleotidyl transferase (TdT) comprises a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1, wherein the terminal deoxynucleotidyl transferase further comprises at least one amino acid substitution or amino acid substitution set at amino acid positions selected from Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, and/or any combinations thereof, wherein the amino acid positions are numbered with reference to SEQ ID NO: 1. In some embodiments, the terminal deoxynucleotidyl transferase comprises amino acid substitution set at amino acid positions Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, wherein the amino acid positions are numbered with reference to SEQ ID NO: 1. In some embodiments, the terminal deoxynucleotidyl transferase further comprises at least one amino acid substitution or amino acid substitution set at amino acid positions selected from Y44K/L, H46K, I47K/E, N58Y, F61Y, Y68K, M72E, T84K/E, I86K/E, V101I, C103K/Y, I109L, D111N/K, E113Y, A119K/E, S128T/A, S143A, E144K, F147Y, M149K, F151Y, S153T, S155E, S159N/K, L174K, C182G/P/D, T211V, E237L/V/R, Q281A/E, S296P, 1310V, C317A, K348N, and A355G/R, and/or any combinations thereof, wherein the amino acid positions are numbered with reference to SEQ ID NO: 1. In some embodiments, the terminal deoxynucleotidyl transferase comprises amino acid substitution set at amino acid positions Y44K/L, H46K, I47K/E, N58Y, F61Y, Y68K, M72E, T84K/E, I86K/E, V101I, C103K/Y, I109L, D111N/K, E113Y, A119K/E, S128T/A, S143A, E144K, F147Y, M149K, F151Y, S153T, S155E, S159N/K, L174K, C182G/P/D, T211V, E237L/V/R, Q281A/E, S296P, 1310V, C317A, K348N, and A355G/R, wherein the amino acid positions are numbered with reference to SEQ ID NO: 1. In some embodiments, the engineered terminal deoxynucleotidyl transferase comprises amino acid substitutions at least at amino acid positions S59, W200, M350, K156, M316, Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, wherein the amino acid positions are numbered with reference to SEQ ID NO: 1. In some embodiments, S59E/A, W200H, M350L/Q, K156E/D, M316L/A, Y44K/L, H46K, I47K/E, N58Y, F61Y, Y68K, M72E, T84K/E, I86K/E, V101I, C103K/Y, I109L, D111N/K, E113Y, A119K/E, S128T/A, S143A, E144K, F147Y, M149K, F151Y, S153T, S155E, S159N/K, L174K, C182G/P/D, T211V, E237L/V/R, Q281A/E, S296P, 1310V, C317A, K348N, and A355G/R, wherein the amino acid positions are numbered with reference to SEQ ID NO: 1.

In some embodiments, the engineered terminal deoxynucleotidyl transferase (TdT) comprises a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, in addition to having amino acid substitutions at least at amino acid positions S59, W200, M350, K156, M316, Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, the engineered terminal deoxynucleotidyl transferase further comprises at least one amino acid substitution or amino acid substitution set at amino acid positions selected from K118, N122, M158, F207, S233, K298, N42, S115, L285, R293, wherein the amino acid positions are numbered with reference to SEQ ID NO: 1.

In some embodiments, the engineered terminal deoxynucleotidyl transferase (TdT) comprises a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, in addition to having amino acid substitutions at least at amino acid positions S59, W200, M350, K156, M316, Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, the engineered terminal deoxynucleotidyl transferase further comprises at least one amino acid substitution or amino acid substitution set at amino acid positions selected from K89, R148, T162, S181, S230, A234, E238, Q239, H290, N300, Q301, Q302, R322, K359, V363, K28, K102, A201, D236, P242, N246, H291, Q292, T306, A375, D380, W385, F52, M88, T91, I104, V120, L121, F129, I157, V180, V191, L194, K196, M210, F226, I245, L247, W248, K250, K251, L287, R347, K366, E368, I382, I54, F165, F173, G192, V193, K361, wherein the amino acid positions are numbered with reference to SEQ ID NO: 1. In some embodiments, the engineered terminal deoxynucleotidyl transferase (TdT) comprises a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least two amino acid substitutions at positions corresponding to residues selected from S59, W200 and M350, wherein the transferase further comprises at least one amino acid substitution or amino acid substitution set at amino acid positions selected from S143, F151, I109, F147, V101, and F61, and/or any combinations thereof, wherein the amino acid positions are numbered with reference to SEQ ID NO: 1.

In some embodiments, the engineered terminal deoxynucleotidyl transferase (TdT) comprises a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least two amino acid substitutions at positions corresponding to residues selected from S59, W200, M350, K156 and M316, wherein the transferase further comprises at least one amino acid substitution or amino acid substitution set at amino acid positions selected from S143, F151, I109, F147, V101, and F61, and/or any combinations thereof, wherein the amino acid positions are numbered with reference to SEQ ID NO: 1.

In some embodiments, disclosed herein is an engineered terminal deoxynucleotidyl transferase comprising a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 2 to 26, wherein the engineered terminal deoxynucleotidyl transferase has increased thermostability and/or increased TdT activity and/or increased protein solubility as compared to the WT TdT. In some embodiment, the engineered terminal deoxynucleotidyl transferase comprises the amino acids 29-389 of the amino acid sequence selected from SEQ ID NO: 2-26. In some embodiments, the terminal deoxynucleotidyl transferase is in a circularly permuted form, wherein the respective portions of the circularly permuted polypeptide have at least 60%, 70%, 80%, 85%, 90%, 91%, 92%, 93%, 94%, 95%, 96%, 97%, 98%, or 99% sequence identity to the respective portions of the amino acids 29-389 of the amino acid sequence selected from SEQ ID NO: 2 to 26.

In some embodiments, the engineered terminal deoxynucleotidyl transferase comprises a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 2 to 26. In some embodiment, the engineered terminal deoxynucleotidyl transferase comprises a polypeptide sequence selected from SEQ ID NO: 2-26.

In some embodiments, the engineered terminal deoxynucleotidyl transferase comprises a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 27 to 49. In some embodiments, the engineered terminal deoxynucleotidyl transferase comprises a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 27 to 49. In some embodiments, the engineered terminal deoxynucleotidyl transferase comprises a polypeptide sequence selected from SEQ ID NO: 27 to 49. In some embodiments, the terminal deoxynucleotidyl transferase is in a circularly permuted form, wherein the respective portions of the circularly permuted polypeptide have at least 60%, 70%, 80%, 85%, 90%, 91%, 92%, 93%, 94%, 95%, 96%, 97%, 98%, or 99% sequence identity to the respective portions of the amino acids 29-389 of the amino acid sequence selected from SEQ ID NO: 27 to 49.

In some embodiments, the engineered TdT according to the present invention may further comprise a tag for improved purification and isolation, expression in a host cell, improved solubility, visualization, detection, functional characterization, enrichment, isolation, cell and/or tissue targeting, and/or cell and/or tissue permeability and/or one or more other beneficial properties. In some embodiments, the engineered TdT according to the present invention is a fusion protein. For example, in some embodiments, the engineered TdT can comprise a protein tag sequence fused to said engineered TdT sequence, wherein said protein tag may be a His tag, a Streptavidin binding protein, Maltose binding protein, a small ubiquitin-like modifier (SUMO), GST tag, HA tag or other protein tag. In some embodiments, a protein tag is fused at N-terminus of the engineered TdT. In some embodiments, a protein tag is fused to the C-terminus of the engineered TdT.

In some embodiments, the engineered TdT according to the present invention may be conjugated to a nucleotide or a nucleotide analog. In some embodiments, the nucleotide is covalently linked to the engineered TdT via a linker. In some embodiments, the linker is selectively cleavable. In some embodiments, the linker is specifically attached to a cysteine residue of the engineered TdT using a sulfhydryl-specific attachment chemistry. In some embodiments, the engineered TdT of the present invention has further amino acid substitutions that result in the engineered TdT having a single cysteine in the polypeptide sequence, wherein said cysteine may further be used to attach said linker.

### Polynucleotides encoding the engineered TdT

The present disclosure also provides polynucleotides encoding the engineered terminal deoxynucleotidyl transferases according to the present invention.

In some embodiments, the polynucleotide is codon-optimised for expression in a target host. In some embodiments, a plasmid comprising the polynucleotide encoding the engineered terminal deoxynucleotidyl transferase according to the present invention is provided. In some embodiments, a cell comprising the terminal deoxynucleotidyl transferase according to the present invention, the polynucleotide encoding the engineered terminal deoxynucleotidyl transferase according to the present invention, or the plasmid comprising the polynucleotide encoding the engineered terminal deoxynucleotidyl transferase according to the present invention is provided.

In some embodiments, the polynucleotide encoding the engineered terminal deoxynucleotidyl transferases comprises a nucleotide sequence selected from SEQ ID NO: 51 to 99.

### Compositions, reaction mixtures, kits

In some embodiments, the present disclosure further provides compositions comprising the engineered terminal deoxynucleotidyl transferase according to the present invention.

In some embodiments, a reaction mixture comprising the engineered terminal deoxynucleotidyl transferase according to the present invention is provided.

In further embodiments, a kit comprising the engineered terminal deoxynucleotidyl transferase according to the present invention is provided.

### Methods of use of engineered TdT

The engineered TdT of the present invention disclosure can be used in various nucleic acid synthesis methods which make use of an enzyme having a terminal deoxynucleotidyl transferase activity. In some embodiments, a method for synthesizing a nucleic acid molecule without template, comprising a step of contacting a nucleic acid primer with at least one nucleotide and an engineered terminal deoxynucleotidyl transferase according to the present invention is provided. In some embodiments, the at least one nucleotide is a synthetic nucleotide, a modified nucleotide or a nucleotide analog, such as, for example, a 3'-O-modified or a 3'-OH-blocked nucleotide.

Advantageously, the engineered terminal deoxynucleotidyl transferase of the present invention can be used at the reaction conditions of elevated reaction temperature, i.e. at a temperature that is higher than 22°C or 37°C. Accordingly, in some embodiments, a method for synthesizing a nucleic acid molecule without template, comprising a step of contacting a nucleic acid primer with at least one nucleotide and the engineered terminal deoxynucleotidyl transferase according to the present invention is provided, wherein the synthesis is performed at temperature higher than 37°C. In some embodiments, the temperature is at least 38°C, at least 39°C, , at least 40°C, at least 41°C, at least 42°C, at least 43°C, at least 44°C, at least 45°C, at least 46°C, at least 47°C, at least 48°C, at least 49°C, at least 50°C, at least 51°C, at least 52°C, at least 53°C, at least 54°C, at least 55°C, at least 56°C, at least 57°C, at least 58°C, at least 59°C, at least 60°C, at least 61°C, at least 62°C, at least 63°C, at least 64°C, at least 65°C, at least 66°C, at least 67°C, at least 68°C, at least 69°C, or at least 70°C. Preferably, the temperature is from about 50°C to about 60°C, from about 60°C to about 70°C, or from about 50°C to about 70°C. In some embodiments, the temperature is 50°C. In some embodiments, the temperature is 60°C. In yet further embodiments, the temperature is 70°C.

Various features and embodiments of the invention are illustrated in the following representative examples, which are intended to be illustrative, and not limiting.

### EXAMPLES

The following Examples, including experiments and results achieved, are provided for illustrative purposes only and are not to be construed as limiting the present invention. Indeed, there are various suitable sources for many of the reagents and equipment described below. It is not intended that the present invention be limited to any particular source for any reagent or equipment item.

### Example 1

### Generation of engineered TdT sequences

The machine learning model as described in WO2023175094A1 has been trained on training data comprising native protein sequences and then used to generate new TdT polymerase sequences with improved properties.

Of the generated TdT sequences with predicted increased thermostability and/or increased TdT activity and/or increased protein solubility, 48 sequences were selected for further testing as described in further examples.

Table 1 provides a summary of the specific amino acid substitutions and their frequency among various engineered TdT variants, wherein the amino acid positions are numbered by reference to the amino acid sequence set forth in SEQ ID NO: 1.

**Table 1**

| aa position | frequency | aa position | frequency | aa position | frequency | aa position | frequency |
|---|---|---|---|---|---|---|---|
| K28E | 9 | M88L | 3 | E144K | 18 | V1911 | 15 |
| K29P | 7 | K89E | 14 | F1471 | 2 | G192E | 3 |
| I30V | 1 | T91I | 4 | F147Y | 25 | G192K | 1 |
| I30L | 1 | T91L | 7 | R148E | 16 | G192L | 8 |
| K38R | 8 | E92K | 5 | R148N | 1 | G192M | 1 |
| N42Q | 1 | E92D | 1 | M149K | 20 | G192T | 1 |
| N42D | 8 | I94L | 1 | M149R | 1 | V193K | 12 |
| N42K | 6 | V101S | 1 | F151Y | 24 | V193G | 2 |
| N42E | 2 | V101T | 2 | S153K | 1 | V193E | 2 |
| Y44L | 6 | V101N | 2 | S153T | 23 | L1941 | 23 |
| Y44N | 1 | V101I | 18 | S155E | 21 | K196R | 5 |
| Y44R | 1 | K102R | 5 | K156E | 14 | K196E | 3 |
| Y44K | 11 | C103K | 20 | K156D | 20 | W200Y | 2 |
| H46R | 3 | C103G | 1 | I157L | 1 | W200H | 19 |
| H46K | 20 | C103Y | 1 | I157V | 12 | W200K | 4 |
| H46E | 1 | I104L | 12 | M158I | 11 | A201Q | 1 |
| I47E | 5 | I104E | 1 | M158K | 9 | A201R | 2 |
| I47K | 16 | I109L | 22 | M158L | 5 | A201K | 8 |
| F52L | 7 | D111K | 5 | S159N | 11 | A201S | 1 |
| F52Y | 1 | D111N | 14 | S159K | 17 | A201E | 2 |
| I54T | 1 | E113Y | 18 | T162N | 9 | F202Y | 4 |
| I54E | 2 | E113K | 1 | T162S | 11 | D205N | 7 |
| I54V | 12 | E113T | 4 | F165Y | 1 | F207N | 1 |
| N58Y | 20 | S115E | 5 | F165L | 8 | F207I | 1 |
| N58D | 1 | S115K | 11 | A171I | 1 | F207T | 1 |
| S59A | 29 | K118R | 1 | A171N | 2 | F207K | 6 |
| S59E | 5 | K118E | 20 | A171E | 1 | F207E | 9 |
| S59Y | 10 | K118L | 1 | A171S | 2 | F207L | 1 |
| F61Y | 22 | A119E | 1 | F173L | 5 | F207Q | 1 |
| Y68N | 1 | A119K | 21 | F1731 | 7 | M210L | 8 |
| Y68K | 17 | V1201 | 14 | L174E | 1 | M210S | 1 |
| M72K | 5 | V120L | 1 | L174K | 21 | M2101 | 3 |
| M72E | 16 | L121K | 13 | V180T | 6 | T211V | 21 |
| F83H | 1 | N122E | 2 | V180K | 4 | T211C | 2 |
| F83Y | 4 | N122K | 14 | V180S | 6 | F226I | 5 |
| F83K | 5 | S128N | 1 | V180N | 3 | F226V | 7 |
| T84E | 12 | S128T | 9 | S181R | 4 | S230T | 8 |
| T84R | 2 | S1281 | 1 | S181K | 5 | S230A | 1 |
| T84K | 5 | S128K | 1 | S181A | 7 | G232S | 3 |
| I86K | 15 | S128A | 13 | C182D | 3 | G232E | 6 |
| I86T | 10 | F129L | 10 | C182P | 16 | S233K | 1 |
| I86E | 4 | F129Y | 4 | C182E | 1 | S233D | 4 |
| I86N | 1 | S143A | 34 | C182G | 2 | S233P | 8 |
| M88I | 7 | E144N | 3 | T184S | 5 | S233N | 1 |
| S233E | 8 | L285T | 1 | Q302R | 2 | K361S | 1 |
| A234E | 16 | L287I | 14 | Q302N | 2 | V363R | 2 |
| A234P | 1 | L287V | 1 | Q302L | 2 | V363Q | 1 |
| A234Q | 1 | H290P | 11 | G304K | 2 | V363K | 7 |
| E235K | 1 | H290E | 3 | T306Q | 5 | V363E | 7 |
| E235N | 1 | H290Y | 3 | T306K | 11 | F364R | 2 |
| E235S | 5 | H290D | 2 | T306D | 2 | F364Y | 1 |
| E235G | 2 | H291L | 3 | K308R | 10 | K366E | 10 |
| D236K | 15 | H291V | 2 | 1310V | 22 | K366P | 2 |
| D236E | 1 | H291D | 2 | M316I | 4 | E368R | 1 |
| E237V | 3 | H291Y | 6 | M316V | 8 | E368K | 9 |
| E237L | 17 | Q292E | 5 | M316L | 15 | A375R | 4 |
| E237R | 1 | Q292K | 6 | M316A | 4 | A375E | 12 |
| E238R | 7 | Q292A | 1 | C317A | 21 | A375K | 7 |
| E238K | 11 | Q292S | 1 | Y319W | 11 | D380E | 17 |
| E238H | 1 | Q292F | 1 | E320D | 6 | 1382V | 15 |
| Q239S | 2 | Q292V | 1 | N321V | 1 | W385K | 1 |
| Q239T | 3 | R293K | 2 | N321Q | 1 | W385H | 6 |
| Q239E | 12 | R293L | 2 | N321Y | 2 | W385Q | 1 |
| P242E | 4 | R293E | 3 | N321E | 1 | W385A | 3 |
| P242K | 6 | R293D | 1 | N321R | 7 | W385E | 1 |
| 1245V | 13 | R293Y | 2 | R322F | 1 | W385S | 3 |
| 1245 L | 1 | R293W | 11 | R322Y | 11 | W385T | 1 |
| N246A | 2 | S296P | 24 | I339L | 6 | | |
| N246E | 3 | S297K | 3 | R347K | 10 | | |
| N246K | 5 | K298L | 8 | K348N | 19 | | |
| N246D | 5 | K298I | 2 | M350I | 3 | | |
| N246T | 1 | K298V | 5 | M350Q | 1 | | |
| L247Y | 10 | N300Q | 1 | M350A | 1 | | |
| W248L | 16 | N300S | 5 | M350G | 1 | | |
| K250E | 14 | N300L | 1 | M350L | 23 | | |
| K251E | 2 | N300P | 4 | A355R | 12 | | |
| K251R | 3 | N300E | 2 | A355G | 10 | | |
| K251Q | 8 | N300G | 1 | A355L | 1 | | |
| Y257S | 3 | N300K | 2 | A355S | 3 | | |
| Y257K | 3 | N300D | 2 | K359E | 1 | | |
| Y257H | 1 | Q301T | 2 | K359R | 8 | | |
| Q281E | 5 | Q301S | 1 | K359L | 7 | | |
| Q281M | 1 | Q301F | 3 | K359I | 1 | | |
| Q281S | 1 | Q301H | 6 | K359T | 3 | | |
| Q281D | 1 | Q301K | 1 | K361N | 1 | | |
| Q281A | 8 | Q301Y | 3 | K361E | 2 | | |
| L285M | 21 | Q302M | 6 | K361R | 3 | | |
| L285C | 2 | Q302I | 6 | K361T | 1 | | |

### Example 2

### Protein solubility analysis and purification

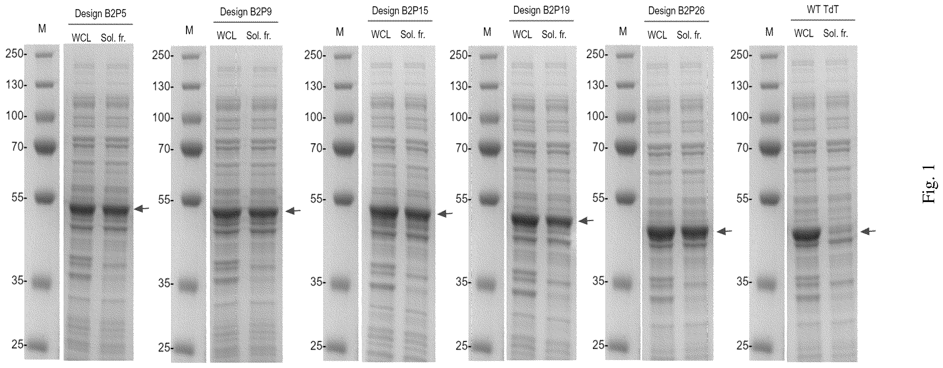

Genes of engineered TdT polymerase enzymes were synthesized (Twist Bioscience) and cloned into T7 RNA polymerase/promoter system vector pET-21. The plasmids carrying these expression cassettes were transformed into electrocompetent E. coli (derived from E. coli B strain) by subjecting cells to 18 000 V/cm electric field. Protein expression was performed in Luria-Bertani media consisting of 2 % tryptone, 1 % yeast extract, 2 % NaCl and 100 µg/mL ampicillin. The cells were grown at 37°C, induced with 0.5 mM IPTG and 0.1 % L-rhamnose at OD 600 = 0.8 and grown for 22 h at 16°C afterwards. Solubility of expressed proteins was analyzed by disrupting cells by sonication and analyzing whole cell lysate (WCL) and soluble fraction (Sol. fr.) samples after centrifugation in SDS-PAGE gels (Fig. 1). Although only a small amount of control protein WT TdT (SEQ ID NO: 1) was present in soluble fraction samples, when compared to WCL samples, the amount of redesigned protein variants remained similar in both of these fractions, indicating that protein solubility was improved dramatically after engineering the TdT sequence. More solubility improvement results are provided in Table 2.

**Table 2.**

| SEQ ID NO | TdT variant | Increase in protein solubility compared to control WT TdT (SEQ ID NO: 1)* |
|---|---|---|
| 2 | R1281B2P15 | +++ |
| 3 | R1281B2P19 | +++ |
| 4 | R1281B2P9 | +++ |
| 5 | R1281B2P5 | +++ |
| 6 | R1281B2P10 | ++ |
| 7 | R1281B2P24 | ++ |
| 8 | R1281B2P2 | +++ |
| 9 | R1281B2P18 | +++ |
| 10 | R1281B2P12 | +++ |
| 11 | R1281B2P21 | +++ |
| 12 | R1281B2P11 | + |
| 13 | R1281B2P13 | +++ |
| 14 | R1281B2P20 | ++ |
| 15 | R1281B2P22 | ++ |
| 16 | R1281B2P1 | + |
| 17 | R1281B2P16 | + |
| 18 | R1281B2P14 | + |
| 19 | R1281B2P17 | + |
| 20 | R1281B2P25 | +++ |
| 21 | R1281B2P26 | +++ |
| 22 | R1281B2P27 | +++ |
| 23 | R1281B2P28 | +++ |
| 24 | R1281B2P4 | +++ |
| 25 | R1281B2P7 | +++ |
| 26 | R1281B2P8 | ++ |

| | | |
|---|---|---|
| * "+" denotes the levels of increased protein solubility of TdT variants relative to the control WT TdT (SEQ ID NO: 1), "+++" denoting the highest increase in solubility over the control TdT. | | |

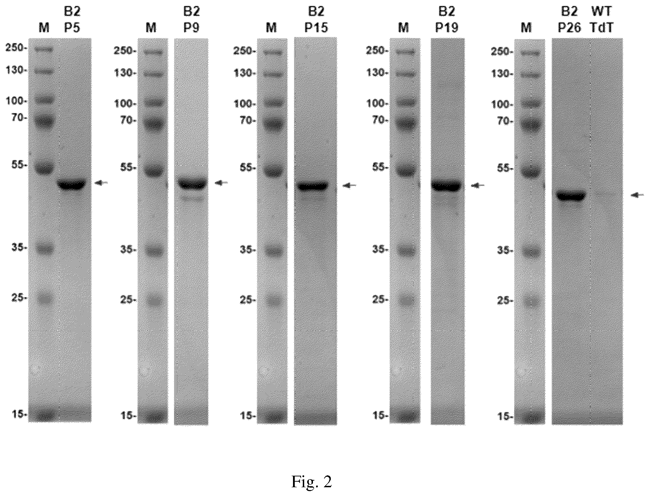

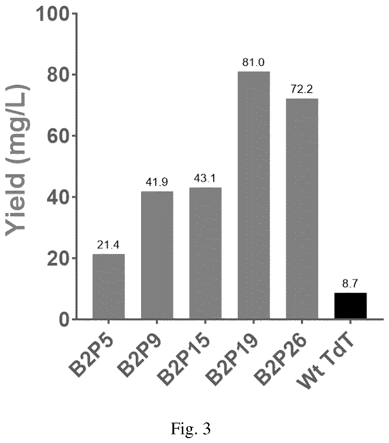

The proteins were expressed with an N-terminal tag containing 6 histidine residues (for this, the protein sequences comprised MGSSHHHHHHSSGENLYFQS (SEQ ID NO: 100) sequence inserted instead of amino acid position 1 of SEQ ID NO: 1 to 49) to facilitate purification (Fig. 2). Purification yields of engineered proteins (in mg of soluble protein per ml of bacterial culture) exceeded the purification yields of control WT TdT by more than 10 times (Fig. 3).

### Example 3

### Protein thermostability measurements

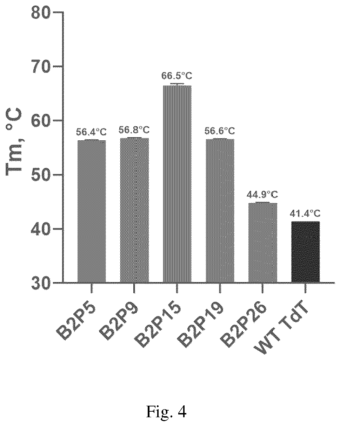

Thermostability of purified TdT variants was determined by measuring protein melting temperatures. Measurements were performed in protein storage buffer (100 mM KOAc (pH 6.8), 2 mM 2-mercaptoethanol, 0.01% (v/v) Tween-20) using GloMelt Dye (Biotium) according to manufacturer's recommendations. The assay was done in a BioRad CFX96 qPCR thermocycler, wheretemperature interval for measurements was 20°C -95°C, temperature was increased in 0.5°C/30s increments. Protein melting curves were generated and analyzed, melting temperatures (Tm) for each TdT design were determined (exemplary results are provided in Fig. 4 and Table 2). Even more than 25°C increase in Tm was observed for the R1281B2P15 variant when compared to WT TdT.

**Table 3**

| SEQ ID NO | TdT variant | Tm, °C | Increase in degrees °C, compared to Tm of control WT TdT (SEQ ID NO: 1) |
|---|---|---|---|
| 1 | WT TdT | 41.4 | |
| 2 | R1281B2P15 | 66.45 | +25.05 |
| 4 | R1281B2P9 | 56.75 | +15.35 |
| 3 | R1281B2P19 | 56.55 | +15.15 |
| 5 | R1281B2P5 | 56.35 | +14.95 |
| 11 | R1281B2P21 | 54.25 | +12.85 |
| 6 | R1281B2P10 | 53 | +11.6 |
| 10 | R1281B2P12 | 52 | +10.6 |
| 12 | R1281B2P11 | 47 | +5.6 |
| 26 | R1281B2P8 | 45.05 | +3.65 |
| 21 | R1281B2P26 | 44.85 | +3.45 |

### Example 4

### Initial screening of designed TdT polymerases ability to add dATP to initiator primer

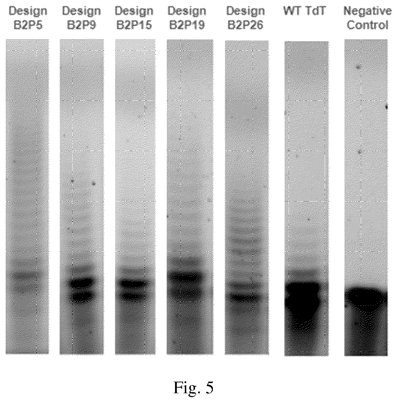

Reaction mixtures containing 10 µM dATP, 0.5 µM single-stranded DNA (ssDNA) initiator primer (5' FAM labeled 5'AATACAACCTCTCA3' (SEQ ID NO: 50), commercial DNA synthesis supplier) and 0.4 µg of purified TdT Polymerase enzymes in 10 µl reaction buffer (0.2 M potassium cacodylate, 0.25 M Tris, 0.05 (v/v) Triton X-100, 1 mM CoCl₂ (pH 7.2 at 25 °C) were prepared. In negative control samples, no TdT polymerase was added. Samples were incubated in 22°C for 5 minutes. Reaction was stopped by adding 2x DNA loading dye (25 mM EDTA, 95% formamide, 0.1% bromophenol blue) in 1:1 ratio and boiling samples in 80°C for 10 min. Samples were analyzed in 20 % PAA gels with 8M Urea. PAA gels were visualized by UV light, detecting the fluorescently labeled initiator primer. Protein activity was determined by visual inspection of the gels by calculating the maximum number of the nucleotides added to the initiator primer. The tested redesigned variants showed improved TdT polymerase activity when compared to WT TdT (Exemplary results are provided in Fig. 5 and Table 3).

**Table 4**

| SEQ ID NO | Variant TdT | Increase in activity at 22°C compared to control WT TdT (SEQ ID NO: 1)* |
|---|---|---|
| 2 | R1281B2P15 | ++ |
| 3 | R1281B2P19 | ++ |
| 4 | R1281B2P9 | +++ |
| 5 | R1281B2P5 | +++ |
| 6 | R1281B2P10 | +++ |
| 7 | R1281B2P24 | ++ |
| 8 | R1281B2P2 | ++ |
| 10 | R1281B2P12 | +++ |
| 11 | R1281B2P21 | ++ |
| 12 | R1281B2P11 | +++ |
| 13 | R1281B2P13 | +++ |
| 14 | R1281B2P20 | +++ |
| 15 | R1281B2P22 | ++ |
| 16 | R1281B2P1 | ++ |
| 17 | R1281B2P16 | + |
| 21 | R1281B2P26 | +++ |
| 26 | R1281B2P8 | + |

| | | |
|---|---|---|
| * - "+" denotes the levels of increased activity of variant TdT proteins relative to the control WT TdT (SEQ ID NO: 1); "+" > 1.05 times; "++" ≥ 1.25 times, "+++" ≥ 1.5 times increase. | | |

### Example 5

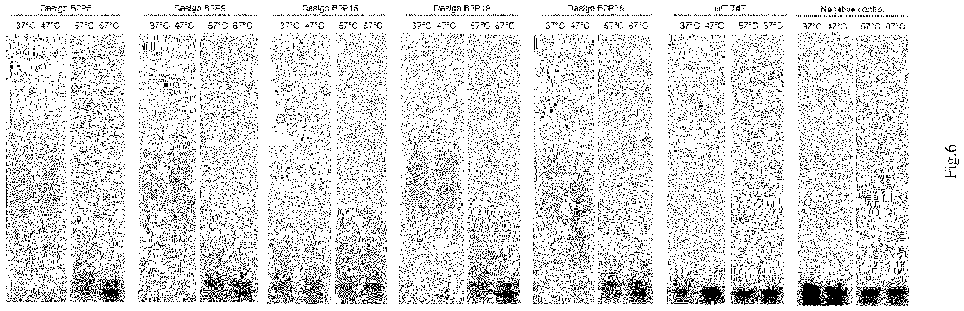

Activity measurements of designed TdT polymerases after incubation in elevated temperatures 2 µl of purified TdT Polymerase enzymes were incubated in 10 µl final volume of reaction buffer (0.2 M potassium cacodylate, 0.25 M Tris, 0.05% (v/v) Triton X-100, 1 mM CoCl₂ (pH 7.2 at 25 °C) for 10 minutes in 37°C, 47°C, 57°C and 67°C temperatures. In negative control samples, no TdT polymerase was added. After incubation, ssDNA initiator primer (5' FAM labeled 5'AATACAACCTCTCA3' (SEQ ID NO: 50), commercial DNA synthesis supplier) and dATP in reaction buffer was added to a final concentration of 0.25 µM and 0.5 µM respectively. DNA extension reaction was performed for 3 min in 22°C temperature. Reaction was stopped by adding 2x DNA loading dye (25 mM EDTA, 95% formamide, 0.1% bromophenol blue) in 1:1 ratio and boiling samples in 80°C for 10 min. Samples were analyzed in 20 % PAA gels with 8M Urea. PAA gels were visualized by UV light, detecting the fluorescently labeled initiator primer. Protein activity was determined by visual inspection of the gels by calculating the maximum number of the nucleotides added to the initiator primer. The redesigned variants retained activity after incubations in higher temperatures better than control WT TdT polymerase (SEQ ID NO: 1) (Fig. 6, Table 4). Design variant R1281B2P15 showed no loss in activity even after incubations of up to 67°C temperatures.

**Table 5.**

| SEQ ID NO | TdT variant | Activity of TdT at 22°C after incubation at indicated elevated temperatures* | | | |
|---|---|---|---|---|---|
| | | 37°C | 47°C | 57°C | 67°C |
| 1 | WT TdT | + | - | - | - |
| 8 | R1281B2P2 | ++ | ++ | ++ | + |
| 5 | R1281B2P5 | +++ | +++ | ++ | + |
| 26 | R1281B2P8 | +++ | + | - | - |
| 4 | R1281B2P9 | +++ | +++ | ++ | + |
| 6 | R1281B2P10 | +++ | +++ | + | - |
| 12 | R1281B2P11 | +++ | +++ | - | - |
| 2 | R1281B2P15 | ++ | ++ | ++ | ++ |
| 3 | R1281B2P19 | +++ | +++ | ++ | + |
| 11 | R1281B2P21 | ++ | ++ | - | - |
| 21 | R1281B2P26 | +++ | +++ | ++ | + |

| | | | | | |
|---|---|---|---|---|---|
| * - activity is provided relative to WT TdT (SEQ ID NO: 1), where higher number of "+" denotes higher activity. | | | | | |

### Example 6

### Activity measurements designed TdT polymerases at elevated temperatures

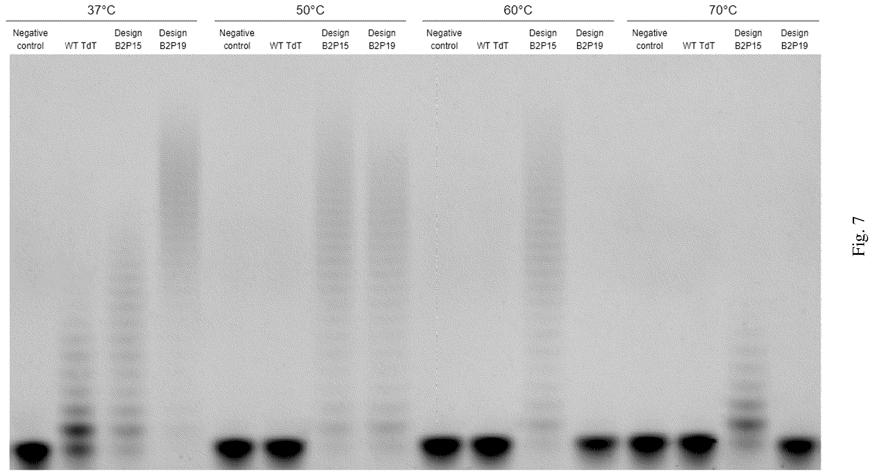

0.1 µg of purified TdT Polymerase variants were incubated in reaction buffer (0.2 M potassium cacodylate, 0.25 M Tris, 0.05% (v/v) Triton X-100, 1 mM CoCl₂ (pH 7.2 at 25 °C) for 10 minutes in 37, 50, 60 and 70°C temperatures. In negative control samples, no TdT polymerase was added. After incubation, ssDNA initiator primer (5' FAM labeled 5'AATACAACCTCTCA3' (SEQ ID NO: 50), commercial DNA synthesis supplier) and dATP in reaction buffer was added to a final concentration of 0.25 µM and 0.5 µM respectively. DNA extension reaction was performed for 3 min in 37°C, 50°C, 60°C and 70°C temperatures. Reaction was stopped by adding 2x DNA loading dye (25 mM EDTA, 95% formamide, 0.1% bromophenol blue) in 1:1 ratio and boiling samples in 80°C for 10 min. Samples were analyzed in 20 % PAA gels with 8M Urea. PAA gels were visualized by UV light, detecting the fluorescently labeled initiator primer. Protein activity was determined by visual inspection of the gels by calculating the maximum number of the nucleotides added to the initiator primer. Engineered TdT designs displayed both increased activity at 37°C and increased retention of activity when performing reactions in elevated temperatures when compared to WT TdT. Designed R1281B2P15 only showed some loss of activity in 70°C degree temperature (Fig. 7, Table 6).

**Table 6**

| SEQ ID NO | TdT variant | Activity of TdT at the indicated temperature, after incubation at the same elevated temperature* | | |
|---|---|---|---|---|
| | | 50°C | 60°C | 70°C |
| 1 | WT TdT | - | - | - |
| 8 | R1281B2P2 | ++ | ++ | + |
| 4 | R1281B2P9 | ++ | + | - |
| 6 | R1281B2P10 | ++ | + | - |
| 12 | R1281B2P11 | + | - | - |
| 11 | R1281B2P21 | ++ | - | - |
| 21 | R1281B2P26 | + | - | - |
| 10 | R1281B2P12 | +++ | - | - |
| 13 | R1281B2P13 | ++ | - | - |
| 14 | R1281B2P20 | +++ | - | - |
| 15 | R1281B2P22 | ++ | - | - |
| 7 | R1281B2P24 | ++ | + | - |

| | | | | |
|---|---|---|---|---|
| * - activity is provided relative to WT TdT (SEQ ID NO: 1). "-" means no activity detected, the number of "+" denotes the levels of detected activity, "+++" meaning more active than "++", and "++" meaning more active than "+". | | | | |

### Example 7

Further embodiments of the present invention are presented here as a clause list:
Clause 1. An engineered terminal deoxynucleotidyl transferase (TdT) comprising a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1.
Clause 2. The engineered terminal deoxynucleotidyl transferase according to clause 1, wherein the engineered terminal deoxynucleotidyl transferase comprises amino acid substitutions at positions corresponding to residues S59, W200 and M350, wherein the positions are numbered by reference to the amino acid sequence set forth in SEQ ID NO: 1.
Clause 3. The engineered terminal deoxynucleotidyl transferase according to clause 1, wherein the engineered terminal deoxynucleotidyl transferase comprises amino acid substitutions at positions corresponding to residues S59, W200, M350, K156 and M316, wherein the positions are numbered by reference to the amino acid sequence set forth in SEQ ID NO: 1.
Clause 4. The engineered terminal deoxynucleotidyl transferase according to clause 1, wherein the engineered terminal deoxynucleotidyl transferase comprises at least two amino acid substitutions at positions corresponding to residues selected from S59, W200 and M350, wherein the positions are numbered by reference to the amino acid sequence set forth in SEQ ID NO: 1.
Clause 5. The engineered terminal deoxynucleotidyl transferase according to clause 1, wherein the engineered terminal deoxynucleotidyl transferase comprises at least two amino acid substitutions at positions corresponding to residues selected from S59, W200, M350, K156 and M316, wherein the positions are numbered by reference to the amino acid sequence set forth in SEQ ID NO: 1.
Clause 6. The engineered terminal deoxynucleotidyl transferase according to any preceding clause, comprising a substitution selected from the group consisting of S59A, S59E, S59Y.
Clause 7. The engineered terminal deoxynucleotidyl transferase according to any preceding clause, comprising a substitution selected from the group consisting of W200H, W200K, W200Y, preferably the substitution is W200H.
Clause 8. The engineered terminal deoxynucleotidyl transferase according to any preceding clause, comprising a substitution selected from the group consisting of M350L, M350Q, M350G, M350A, M350I, preferably the substitution is M350L.
Clause 9. The engineered terminal deoxynucleotidyl transferase according to any preceding clause, comprising a substitution selected from the group consisting of K156E, K156D.
Clause 10. The engineered terminal deoxynucleotidyl transferase according to any preceding clause, comprising a substitution selected from the group consisting of M316L, M316A, M316V, M316I.
Clause 11. The engineered terminal deoxynucleotidyl transferase according to any one of preceding clauses having at least 60%, 70%, 80%, 85%, 90%, 91%, 92%, 93%, 94%, 95%, 96%, 97%, 98%, or 99% sequence identity to a sequence selected from SEQ ID NO: 2 to 26.
Clause 12. The engineered terminal deoxynucleotidyl transferase according to any one of preceding clauses, having at least 60%, 70%, 80%, 85%, 90%, 91%, 92%, 93%, 94%, 95%, 96%, 97%, 98%, or 99% sequence identity to a sequence selected from SEQ ID NO: 27 to 49.
Clause 13. The engineered terminal deoxynucleotidyl transferase any one of clauses 1 to 10, wherein the transferase further comprises at least one amino acid substitution or amino acid substitution set at amino acid positions selected from Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, and/or any combinations thereof, wherein the amino acid positions are numbered with reference to SEQ ID NO: 1.
Clause 14. The engineered terminal deoxynucleotidyl transferase of clause 13, wherein the transferase comprises amino acid substitutions at least at amino acid positions S59, W200, M350, K156, M316, Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, wherein the amino acid positions are numbered with reference to SEQ ID NO: 1.
Clause 15. The engineered terminal deoxynucleotidyl transferase of clause 14, wherein the transferase further comprises at least one amino acid substitution or amino acid substitution set at amino acid positions selected from K118, N122, M158, F207, S233, K298, N42, S115, L285, R293, wherein the amino acid positions are numbered with reference to SEQ ID NO: 1.
Clause 16. The engineered terminal deoxynucleotidyl transferase according to any one of clauses 13 to 15, wherein the transferase further comprises at least one amino acid substitution or amino acid substitution set at amino acid positions selected from K89, R148, T162, S181, S230, A234, E238, Q239, H290, N300, Q301, Q302, R322, K359, V363, K28, K102, A201, D236, P242, N246, H291, Q292, T306, A375, D380, W385, F52, M88, T91, I104, V120, L121, F129, I157, V180, V191, L194, K196, M210, F226, I245, L247, W248, K250, K251, L287, R347, K366, E368, I382, I54, F165, F173, G192, V193, K361, wherein the amino acid positions are numbered with reference to SEQ ID NO: 1.
Clause 17. The engineered terminal deoxynucleotidyl transferase of clause 4 or 5, wherein the transferase further comprises at least one amino acid substitution or amino acid substitution set at amino acid positions selected from S143, F151, I109, F147, V101, and F61, and/or any combinations thereof, wherein the amino acid positions are numbered with reference to SEQ ID NO: 1.
Clause 18. An engineered terminal deoxynucleotidyl transferase comprising a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 2 to 26.
Clause 19. The engineered terminal deoxynucleotidyl transferase according to clause 18, comprising a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 2 to 26.
Clause 20. The engineered terminal deoxynucleotidyl transferase according to clause 19, comprising a polypeptide sequence selected from SEQ ID NO: 2-26.
Clause 21. An engineered terminal deoxynucleotidyl transferase comprising a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 27 to 49.
Clause 22. The engineered terminal deoxynucleotidyl transferase according to clause 21, comprising a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 27 to 49.
Clause 23. The engineered terminal deoxynucleotidyl transferase according to clause 22, comprising a polypeptide sequence selected from SEQ ID NO: 27 to 49.
Clause 24. A composition comprising the engineered terminal deoxynucleotidyl transferase according to any of the preceding clauses.
Clause 25. A reaction mixture comprising the engineered terminal deoxynucleotidyl transferase according to any of clauses 1 to 23.
Clause 26. A kit comprising the engineered terminal deoxynucleotidyl transferase according to any of clauses 1 to 23.
Clause 27. A polynucleotide encoding the terminal deoxynucleotidyl transferase according to any of clauses 1 to 23.
Clause 28. A polynucleotide according to clause 27, wherein the polynucleotide is codon-optimised for expression in a target host.
Clause 29. A plasmid comprising the polynucleotide of clause 28.
Clause 30. A cell comprising the terminal deoxynucleotidyl transferase according to any one of clauses 1 to 23, the polynucleotide according to clauses 27 or 28, or the plasmid according to clause 29.
Clause 31. A method for synthesizing a nucleic acid molecule without template, comprising a step of contacting a nucleic acid primer with at least one nucleotide and the engineered terminal deoxynucleotidyl transferase as defined in any of preceding clauses.
Clause 32. The method according to clause 31, wherein the synthesis is performed at temperature of at least 50°C.
Clause 33. The method according to clause 31, wherein the synthesis is performed at temperature of at least 60°C.
Clause 34. The method according to clause 31, wherein the synthesis is performed at temperature of at least 70°C.

### References

Hoose A, Vellacott R, Storch M, Freemont PS, Ryadnov MG. DNA synthesis technologies to close the gene writing gap. Nat Rev Chem. 2023;7(3):144-161. doi: 10.1038/s41570-022-00456-9. Epub 2023 Jan 23. Erratum in: Nat Rev Chem. 2023 Aug;7(8):590. PMID: 36714378; PMCID: PMC9869848.
Chua JPS, Go MK, Osothprarop T, Mcdonald S, Karabadzhak AG, Yew WS, Peisajovich S, Nirantar S. Evolving a Thermostable Terminal Deoxynucleotidyl Transferase. ACS Synth Biol. 2020 Jul 17;9(7):1725-1735. doi: 10.1021/acssynbio.0c00078. Epub 2020 Jun 23. PMID: 32497424.

## Claims

1. An engineered terminal deoxynucleotidyl transferase (TdT) comprising a polypeptide sequence having at least 60%, 70%, 80%, 85%, 90%, 91%, 92%, 93%, 94%, 95%, 96%, 97%, 98%, or 99% sequence identity to a sequence SEQ ID NO: 1, or a functional fragment thereof, wherein the engineered terminal deoxynucleotidyl transferase comprises at least one amino acid substitution or amino acid substitution set in its polypeptide sequence, wherein the at least one amino acid substitution or amino acid substitution set comprises an amino acid substitution or an amino acid substitution set(s) at amino acid positions selected from S59, W200, M350, K156 and M316 and/or any combinations thereof, wherein the positions are numbered by reference to the amino acid sequence set forth in SEQ ID NO: 1.

2. The engineered terminal deoxynucleotidyl transferase according to claim 1, wherein the engineered terminal deoxynucleotidyl transferase comprises:
a) amino acid substitutions at positions corresponding to residues S59, W200 and M350, or
b) amino acid substitutions at positions corresponding to residues S59, W200, M350, K156 and M316.

3. The engineered terminal deoxynucleotidyl transferase according to claim 1, wherein the engineered terminal deoxynucleotidyl transferase comprises,
a) at least two amino acid substitutions at positions corresponding to residues selected from S59, W200 and M350, or
b) at least two amino acid substitutions at positions corresponding to residues selected from S59, W200, M350, K156 and M316,
wherein the positions are numbered by reference to the amino acid sequence set forth in SEQ ID NO: 1.

4. The engineered terminal deoxynucleotidyl transferase according to any one of preceding claims having at least 60%, 70%, 80%, 85%, 90%, 91%, 92%, 93%, 94%, 95%, 96%, 97%, 98%, or 99% sequence identity to a sequence selected from SEQ ID NO: 2 to 26.

5. The engineered terminal deoxynucleotidyl transferase according to any one of preceding claims, having at least 60%, 70%, 80%, 85%, 90%, 91%, 92%, 93%, 94%, 95%, 96%, 97%, 98%, or 99% sequence identity to a sequence selected from SEQ ID NO: 27 to 49.

6. The engineered terminal deoxynucleotidyl transferase according to any one of the claims 1 to 3, wherein the transferase further comprises at least one amino acid substitution or amino acid substitution set at amino acid positions selected from Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, and/or any combinations thereof, wherein the amino acid positions are numbered with reference to SEQ ID NO: 1.

7. The engineered terminal deoxynucleotidyl transferase of claim 6, wherein the transferase comprises amino acid substitutions at least at amino acid positions S59, W200, M350, K156, M316, Y44, H46, I47, N58, F61, Y68, M72, T84, I86, V101, C103, I109, D111, E113, A119, S128, S143, E144, F147, M149, F151, S153, S155, S159, L174, C182, T211, E237, Q281, S296, I310, C317, K348, and A355, wherein the amino acid positions are numbered with reference to SEQ ID NO: 1.

8. The engineered terminal deoxynucleotidyl transferase of claim 6 or 7,
wherein the transferase further comprises at least one amino acid substitution or amino acid substitution set at amino acid positions selected from K118, N122, M158, F207, S233, K298, N42, S115, L285, R293, and/or
wherein the transferase further comprises at least one amino acid substitution or amino acid substitution set at amino acid positions selected from K89, R148, T162, S181, S230, A234, E238, Q239, H290, N300, Q301, Q302, R322, K359, V363, K28, K102, A201, D236, P242, N246, H291, Q292, T306, A375, D380, W385, F52, M88, T91, I104, V120, L121, F129, I157, V180, V191, L194, K196, M210, F226, I245, L247, W248, K250, K251, L287, R347, K366, E368, I382, I54, F165, F173, G192, V193, K361,
wherein the amino acid positions are numbered with reference to SEQ ID NO: 1.

9. The engineered terminal deoxynucleotidyl transferase according to claim 3, wherein the transferase further comprises at least one amino acid substitution or amino acid substitution set at amino acid positions selected from S143, F151, I109, F147, V101, and F61, and/or any combinations thereof, wherein the amino acid positions are numbered with reference to SEQ ID NO: 1.

10. An engineered terminal deoxynucleotidyl transferase comprising:
a) a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 2 to 26,
b) a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 2 to 26, or
c) a polypeptide sequence selected from SEQ ID NO: 2-26.

11. An engineered terminal deoxynucleotidyl transferase comprising:
a) a polypeptide sequence having at least 60%, 70%, 80%, 85%, 90%, 91%, 92%, 93%, 94%, 95%, 96%, 97%, 98%, or 99% sequence identity to amino acids 29-389 of the amino acid sequence selected from SEQ ID NO: 27 to 49,
b) a polypeptide sequence having at least 60%, 70%, 80%, 85%, 90%, 91%, 92%, 93%, 94%, 95%, 96%, 97%, 98%, or 99% sequence identity to a sequence selected from SEQ ID NO: 27 to 49, or
c) a polypeptide sequence selected from SEQ ID NO: 27 to 49.

12. A composition, a reaction mixture, or a kit comprising the engineered terminal deoxynucleotidyl transferase according to any of claims 1 to 11.

13. A polynucleotide encoding the terminal deoxynucleotidyl transferase according to any of claims 1 to 11.

14. A method for synthesizing a nucleic acid molecule without template, comprising a step of contacting a nucleic acid primer with both at least one nucleotide and the engineered terminal deoxynucleotidyl transferase as defined in any of preceding claims.

15. The method according to claim 14, wherein the synthesis is performed at temperature of:
a) at least 50°C,
b) at least 60°C, or
c) at least 70°C.
